(19) Europäisches Patentamt
European Patent Office
Office européen des brevets

(11) Veröffentlichungsnummer : **0 325 974 B1**

(12) **EUROPÄISCHE PATENTSCHRIFT**

(45) Veröffentlichungstag der Patentschrift :
**08.04.92 Patentblatt 92/15**

(51) Int. Cl.$^5$ : **C07D 487/22,** C08K 5/34,
// (C07D487/22, 251:00,
251:00, 241:00, 241:00)

(21) Anmeldenummer : **89100617.3**

(22) Anmeldetag : **14.01.89**

# (54) Heterocyclen auf Piperazinopiperazinbasis und deren Verwendung.

Die Akte enthält technische Angaben, die nach dem Eingang der Anmeldung eingereicht wurden und die nicht in dieser Patentschrift enthalten sind.

(30) Priorität : **23.01.88 DE 3801944**

(43) Veröffentlichungstag der Anmeldung :
**02.08.89 Patentblatt 89/31**

(45) Bekanntmachung des Hinweises auf die Patenterteilung :
**08.04.92 Patentblatt 92/15**

(84) Benannte Vertragsstaaten :
**CH DE FR GB IT LI**

(56) Entgegenhaltungen :
**EP-A- 0 213 570**
**EP-A- 0 272 589**
**EP-A- 0 272 590**
**FR-A- 2 291 203**

(73) Patentinhaber : **BASF Aktiengesellschaft**
**Carl-Bosch-Strasse 38**
**W-6700 Ludwigshafen (DE)**

(72) Erfinder : **Neumann, Peter, Dr.**
**Poststrasse 28**
**W-6800 Mannheim 31 (DE)**
Erfinder : **Aumueller, Alexander, Dr.**
**An der Marlach 5**
**W-6705 Deidesheim (DE)**
Erfinder : **Trauth, Hubert**
**Milanstrasse 5**
**W-6724 Dudenhofen (DE)**

**EP 0 325 974 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, daß Polyalkylpiperidinderivate und sterisch gehinderte Phenole organische Polymere von Zerstörung durch Licht und wärme schützen. So werden beispielsweise in der EP-A-O 213 570 Polyalkylpiperidin-substituierte Glykolurilderivate als Lichtschutzmittel insbesondere für Kunststoffe empfohlen.

Unbefriedigend ist oft die Verträglichkeit dieser Piperidinderivate mit Polyolefinen und anderen Kunststoffen, die Dauer der Schutzwirkung, die Eigenfarbe der Substanzen sowie die Neigung zur Flüchtigkeit und die thermische Zersetzung der Stabilisatoren beim Einarbeiten in Polymere bei erhöhter Temperatur.

Der Erfindung liegt die Aufgabe zugrunde, neue Stabilisatoren zur Verfügung zu stellen, welche die vorstehenden Nachteile vermeiden bzw. nicht aufweisen.

Die Aufgabe wird mit Hilfe der neuen Heterocyclen der vorliegenden Erfindung gelöst.

Dementsprechend betrifft die Erfindung Heterocyclen der allgemeinen Formel (I)

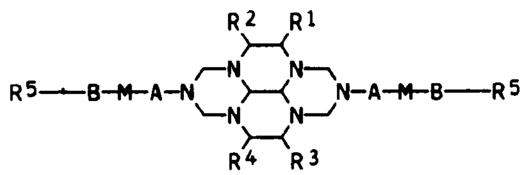

(I),

in der

$R^1$, $R^2$, $R^3$ und $R^4$ unabhängig voneinander für Wasserstoff, $C_1$- bis $C_{22}$-Alkyl oder $C_5$- oder $C_6$-Cycloalkyl oder $R^1$ und $R^2$ sowie $R^3$ und $R^4$ jeweils zusammen für eine Tri- oder Tetramethylengruppe,

A für eine direkte Bindung, Methylen oder Ethylen der Formel $-CH_2-CH_2-$,

M für eine Gruppierung der Formel

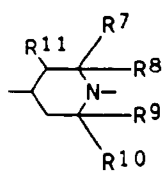

wobei M sowohl mit dem Stickstoff als auch mit dem Kohlenstoff an A gebunden sein kann und worin

$R^7$, $R^8$, $R^9$ und $R^{10}$ unabhängig voneinander $C_1$- bis $C_4$-Alkyl oder

$R^7$ und $R^8$ und/oder $R^9$ und $R^{10}$ zusammen eine Tetra- oder Pentamethylengruppe und

$R^{11}$ Wasserstoff oder $C_1$- bis $C_4$-Alkyl bedeuten,

B für eine direkte Bindung, eine $C_1$- bis $C_{22}$-Alkylengruppe, eine $C_7$- bis $C_{16}$-Phenylalkylengruppe oder für eine durch Carbonyl, Carbonamid oder Carbonester unterbrochene $C_3$- bis $C_{22}$-Alkylengruppe und

$R^5$ für wasserstoff, Cyan oder Hydroxy oder

M-B-$R^5$ auch für eine Gruppe der Formeln

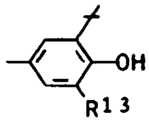 , 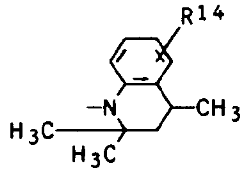 , 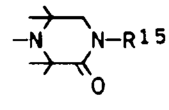 ,

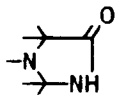 oder 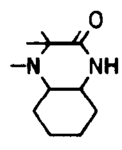 steht, worin

$R^{13}$ $C_1$- bis $C_4$-Alkyl,

$R^{14}$ Wasserstoff, $C_1$- bis $C_4$-Alkyl oder $C_1$- bis $C_4$-Alkoxy und
$R^{15}$ $C_1$- bis $C_{12}$-Alkyl bedeuten.

Die erfindungsgemäßen Verbindungen besitzen außerordentlich gute stabilisierende Eigenschaften, haben keine Eigenfarbe, sind mit organischen Polymeren gut verträglich und zeigen einen niedrigen Dampfdruck.

Bevorzugt sind Heterocyclen (I), in denen $R^7$, $R^8$, $R^9$ und $R^{10}$ Methyl und $R^{11}$ wasserstoff sind.

Die Alkylreste für $R^1$, $R^2$, $R^3$, $R^4$ und $R^{15}$ können sowohl verzweigt als auch unverzweigt sein. Im einzelnen sind z.B. zu nennen: Methyl, Ethyl, Propyl, i-Propyl, Butyl, iso-Butyl, Pentyl, Hexyl, Octyl, Decyl, Lauryl, Tetradecyl, Hexadecyl und Stearyl. $R^1$ bis $R^4$ stehen vorzugsweise für Methyl und insbesondere für wasserstoff.

Bevorzugt sind außerdem Verbindungen der Formel (I), in denen sowohl $R^1$ und $R^2$ als auch $R^3$ und $R^4$ jeweils zusammen eine Tetramethylengruppe bilden.

Als $C_1$-$C_4$-Alkoxy für $R^{14}$ sind im einzelnen z.B. Methoxy, Ethoxy, Propoxy und Buroxy zu nennen.

Bevorzugt sind für B die direkte Bindung, Methylen und Ethylen der Formel $-CH_2-CH_2-$.

Als $C_1$-$C_4$-Alkyl sind für $R^{13}$ und $R^{14}$ z.B zu nennen: Methyl, Ethyl, Propyl, i-Propyl, Butyl, i-Butyl und tert Butyl. Für $R^{13}$ ist Methyl und insbesondere tert.-Butyl bevorzugt.

Die Verbindungen der allgemeinen Formel (I) lassen sich durch Reaktion von Formaldehyd, Paraformaldehyd oder Trioxan und Piperazinopiperazinen der allgemeinen Formel (II) mit Verbindungen der allgemeinen Formeln (III) und (IV) bevorzugt in Gegenwart von Ionenaustauschern als Katalysator darstellen.

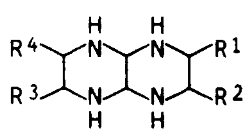

(II)

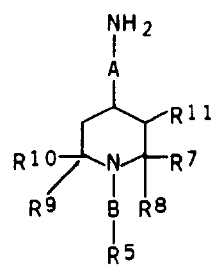

(III)

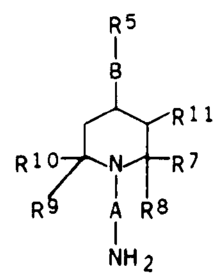

(IV)

Lösungsmittel ist dabei wasser oder ein organisches Lösungsmittel. Bevorzugt sind hierfür Alkohole, insbesondere Methanol und ganz besonders Ethanol.

Besonders vorteilhaft ist die Verwendung von Kationenaustauschern, insbesondere von Sulfonatgruppen enthaltenden unlöslichen Polymeren, die mit Alkalimetallionen, z.B. Natriumionen beladen sind.

Einsetzbar sind auch Gemische aus Verbindungen der allgemeinen Formeln (III) und (IV). Man erhält dann dementsprechend Gemische aus Verbindungen der allgemeinen Formel (II).

Die Ausgangsmaterialien der allgemeinen Formel (II) sind z.B. in Rec. Trav. Chim. Pays-Bas 98, 326 (1979) und der US-PS 2 345 237 beschrieben.

Verbindungen der allgemeinen Formel (I), die noch reaktive Gruppen tragen, können nach Standardverfahren der organischen Chemie, beispielsweise durch Alkylierung, Acylierung oder Cyanmethylierung in neue Verbindungen der allgemeinen Formel (I) umgewandelt werden.

Die erfindungsgemäßen Verbindungen können in Form der freien Basen oder als Salze vorliegen. Geeignete Anionen stammen z. B. von anorganischen Säuren und insbesondere organischen Carbonsäuren sowie organischen Sulfonsäuren.

Anorganische Anionen sind z. B.: Chlorid, Bromid, Sulfat, Methosulfat, Tetrafluoroborat, Phosphat oder Rhodanid.

Carbonsäure-Anionen sind beispielsweise: Formiat, Acetat, Propionat, Hexanoat, Cyclohexanoat, Lactat, Stearat, Dodecylbenzoat, Benzoat, Acrylat, Methacrylat, Zitrat, Malonat oder Succinat sowie Anionen von Polycarbonsäuren mit bis zu 3000 COOH-Gruppen.

Sulfonsäure-Anionen sind beispielsweise Benzolsulfonat oder Tosylat.

Die erfindungsgemäßen Verbindungen eignen sich zum Stabilisieren von organischem Material, speziell von Kunststoffen, gegen den Abbau durch Licht und wärme. Sie sind auch wirksam als Metalldesaktivator. Sie werden den zu stabilisierenden Kunststoffen in einer Konzentration von 0,01 bis 5 Gew.%, vorzugsweise von 0,02 bis 2 Gew.% vor, während oder nach der Polymerbildung zugesetzt.

Zur Vermischung der erfindungsgemäßen Verbindungen mit den zu stabilisierenden Kunststoffen können alle bekannten Vorrichtungen und Methoden zum Einmischen von Stabilisierungsmitteln oder anderen Zusätzen in Polymere angewandt werden.

Die durch eine der erfindungsgemäßen Verbindungen stabilisierten Kunststoffe können gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die den Kunststoffen neben den erfindungsgemäßen Verbindungen zugesetzt werden können, sind z.B. Verbindungen auf der Basis sterisch gehinderter Phenole oder Schwefel oder Phosphor enthaltende Costabilisatoren.

Als derartige phenolische Antioxidationsmittel seien beispielsweise 2,6-di-tert.-butyl-4-methylphenol, n-Octadecyl-β-(3,5-di-tert.-butyl-4-hydro-xyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxyphenyl)-propionyloxy-ethyl]-isocyanurat, 1,3,5-Tris-(2,6,di-methyl-3-hydroxy-4-tert.-butylbenzyl)-isocyanurat und Pentaerythrit-tetrakis-[β-(3,5-di-tert.-butyl-4-hydroxy-phenyl)-propionat] erwähnt.

Als phosphorhaltige Antioxidantien seien beispielsweise Tris-(nonyl-phenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4′-biphenylendiphosphit erwähnt.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) und Pentaerythrittetrakis-(β-hexylthiopropionat) erwähnt.

Weitere Antioxidantien und Lichtstabilisatoren, die zusammen mit den erfindungsgemäßen Verbindungen verwendet werden können, sind z.B. 2-(2′-Hydroxyphenyl)-benztriazole, 2-Hydroxybenzophenone, Arylester von Hydroxybenzoesäuren, α-Cyanozimtsäurederivate, Nickelverbindungen oder Oxalsäuredianilide.

Als organische Polymere, die durch die erfindungsgemäßen Verbindungen stabilisiert werden können, seien beispielsweise genannt:

Polymere von Mono- und Diolefinen, wie z. B. Polyethylen niedriger oder hoher Dichte, lineares Polyethylen niedriger Dichte, Polypropylen, Polyisobutylen, Polybuten-1, Polyisopren, Polybutadien sowie Copolymerisate von Mono- oder Diolefinen oder Mischungen der genannten Polymeren;

Copolymerisate von Mono- oder Diolefinen mit anderen Vinylmonomeren wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polysytrol;

Copolymere von Styrol oder α-Methylstyrol mit Dienen oder Acrylderivaten, wie z.B. Styrol-Butadten, Styrol-Acrylnitril, Styrol-Ethylmethacrylat, Styrol-Butadien-Ethylacrylat, Styrol-Acrylnitril-Methacrylat;

ABS-, MBS- oder ähnliche Polymere;

Halogenhaltige Polymere, wie z. B. Polyvinylchlorid, Polyvinylfluorid, Polyvinylidenfluorid sowie deren Copolymere;

Polymere die sich von α,β-ungesättigten Säuren und deren Derivaten ableiten, wie Polyacrylate und Polymethacrylate, Polyacrylamide und Polyacrylnitrile.

Polymere, die sich von ungesättigten Alkoholen und Aminen bzw. deren Acrylderivaten oder Acetalen ableiten, wie Polyvinylalkohol oder Polyvinylacetat;

Polyurethane, Polyamide, Polyharnstoffe, Polyester, Polycarbonate, Polysulfone, Polyethersulfone und Polyetherketone.

Weitere organische Polymere, die mit den erfindungsgemäßen Verbindungen stabilisiert werden können, stellen Industrielackierungen dar. Unter diesen sind Einbrennlackierungen, unter diesen wiederum Fahrzeuglackierungen, vorzugsweise Zweischichtlackierungen, besonders hervorzuheben.

Auch hier können zusätzlich die bereits aufgeführten Antioxidantien und Lichtschutzmittel verwendet werden.

Die erfindungsgemäßen Verbindungen können in fester oder gelöster Form dem Lack zugesetzt werden. Ihre gute Löslichkeit in Lacksystemen ist dabei von besonderem Vorteil.

Bevorzugt ist die Verwendung der erfindungsgemäßen Verbindungen zur Stabilisierung von Polyolefinen, vorzugsweise von Ethylen- und Propylenpolymerisaten, Polyamiden und Polyurethanen sowie von Lacken.

Die Erfindung wird durch die nachstehenden Beispiele weiter erläutert

Beispiel 1

56,8 g 1,4,5,8-Tetraazadecalin, 48 g Paraformaldehyd, 124,8 g 2,2,6,6-Tetramethyl-4-aminopiperidin und 20 g Lewatit® S100 wurden in 400 ml Ethanol 6h bei Raumtemperatur gerührt. Der Niederschlag wurde durch Erhitzen in Lösung gebracht, die Lösung heiß filtriert und im Eisbad gekühlt. Nach dem Absaugen isolierte man die Verbindung (V) in Form farbloser Kristalle vom Schmelzpunkt 233-235°C. Nach dem Umkristallisieren aus Hexan stieg der Schmelzpunkt auf 236-238°C.

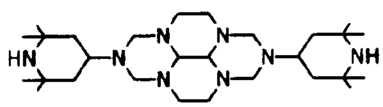

(V)

Beispiel 2

17 g 2,6-Dimethyl-1,4,5,8-tetraazadecalin, 12 g Paraformaldehyd, 31,2 g 2,2,6,6-Tetramethyl-4-aminopiperidin und 5 g Lewatit® S100 wurden in 100 ml Ethanol 6h bei Raumtemperatur gerührt. Man filtrierte vom Katalysator ab und engte fast bis zur Trockne ein. Der Rückstand wurde mit wasser zweimal heiß ausgekocht, getrocknet und aus Toluol umkristallisiert. Man isolierte die Verbindung (VI) in Form farbloser Kirstalle vom Schmelzpunkt 220-224°C.

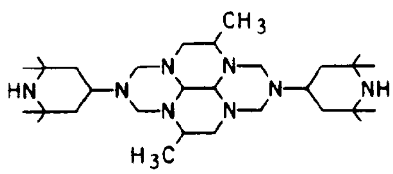

(VI)

Beispiel 3

7,51 g Perhydro-5,6,11,12-tetraaza-tetracen, 3,6 g Parafomaldehyd, 9,36 g 2,2,6,6-Tetramethyl-4-aminopiperidin und 2 g Lewatit® S100 wurden in 50 ml Ethanol 5 h gekocht. Man filtrierte, engte ein, kochte in 150 ml n-Hexan auf und saugte ab. Man erhielt die Verbindung (VII) in Form leicht bräunlicher Kristalle vom Schmelzpunkt 232-235°C.

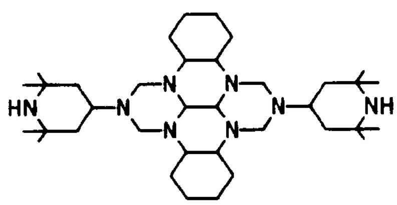

(VII)

Beispiel 4

14,2 g 1,4,5,8-Tetraazadecalin, 12 g Paraformaldehyd, 40 g 2,2,6,6-Tetramethyl-1-[2-aminoethyl]-4-hydroxy-piperidin und 5,1 g Lewatit® S100 wurden in 250 ml Ethanol 9,5 h gekocht. Der ausgefallene Niederschlag wurde abgesaugt, mit Ethanol gewaschen und aus DMF umkristallisiert. Man isolierte Verbindung (VIII) als farblosen Feststoff vom Schmelzpunkt 293-294°C.

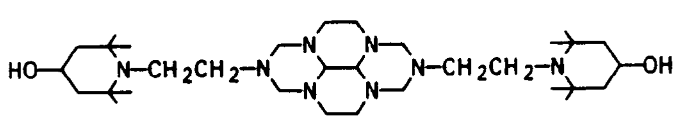

(VIII)

Beipiel 5

4,7 g 1,4,5,8-Tetraazadecalin, 4 g Paraformaldehyd, 15,7 g 4-Aminomethyl-2,6-di-t-butyl-phenol und 1,7

g Lewatit® S100 wurden 4,5 h in 250 ml Ethanol gekocht. Der ausgefallene Niederschlag wurde abgesaugt und aus i-Butanol umkristallisiert. Man isolierte die Verbindung (IX) als farblosen Feststoff vom Schmelzpunkt 249°C.

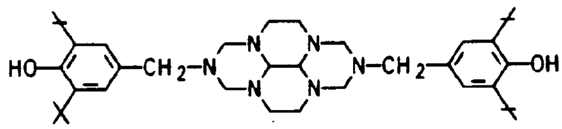

(IX)

Beispiel 6

5 ml mit Kaliumcarbonat gesättigtes Ethanol, 6 g Paraformaldehyd, 0,46 g Kaliumcarbonat und 17 g Acetoncyanhydrin werden 2 h bei 25-30°C gerührt. Das Gemisch wurde mit Phosphorsäure auf pH = 6 eingestellt. Dann wurden 25,1 g des Produkts aus Beispiel 1 und 50 ml Ethanol zugegeben und das Gemisch 4 h auf Rückflußtemperatur gehalten. Dann wurde abgesaugt, der Rückstand mit Wasser ausgekocht und getrocknet. Man isolierte die Verbindung (X) als farblosen Feststoff vom Schmelzpunkt 270-273°C.

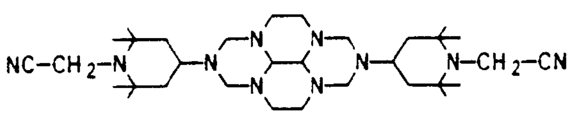

(X)

Beispiel 7

6,2 g 1,4,5,8-Tetraazadecalin, 29 g 1-[2-Aminoethyl]-6-ethoxy-1,2,3,4-tetrahydro-2,2,4-trimethylchinolin, 5,3 g Paraformaldehyd und 2,5 g Lewatit® S100 wurden 7 h in 200 ml Ethanol am Rückfluß gekocht. Der Rückstand wurde abgesaugt und zweimal aus n-Butanol umkristallisiert. Man erhielt Verbindung (XI) als farblosen Feststoff vom Schmelzpunkt 184-185°C.

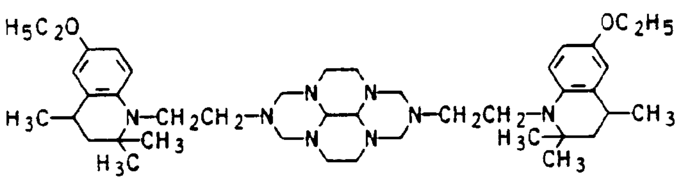

(XI)

**Patentansprüche**

1. Heterocyclen der allgemeinen Formel (I)

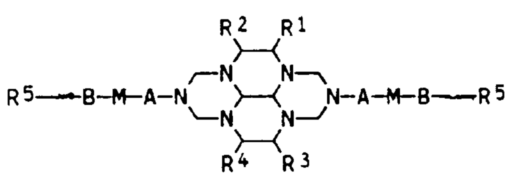

(I),

in der
$R^1$, $R^2$, $R^3$ und $R^4$ unabhängig voneinander für Wasserstoff, $C_1$- bis $C_{22}$-Alkyl oder $C_5$- oder $C_6$-Cycloalkyl oder $R^1$ und $R^2$ sowie $R^3$ und $R^4$ jeweils zusammen für eine Tri- oder Tetramethylengruppe,
A für eine direkte bindung, Methylen oder Ethylen der Formel $-CH_2-CH_2-$,
M für eine Gruppierung der Formel

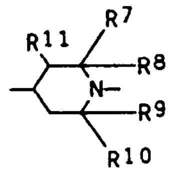,

wobei M sowohl mit dem Stickstoff als auch mit dem Kohlenstoff an A gebunden sein kann und worin
$R^7$, $R^8$, $R^9$ und $R^{10}$ unabhängig voneinander $C_1$- bis $C_4$-Alkyl oder
$R^7$ und $R^8$ und/oder $R^9$ und $R^{10}$ zusammen eine Tetra- oder Pentamethylengruppe und
$R^{11}$ Wasserstoff oder $C_1$- bis $C_4$-Alkyl bedeuten,
B für eine direkte bindung, eine $C_1$- bis $C_{22}$-Alkylengruppe, eine $C_7$- bis $C_{16}$-Phenylalkylengruppe oder für eine durch Carbonyl, Carbonamid oder Carbonester unterbrochene $C_3$- bis $C_{22}$-Alkylengruppe und
$R^5$ für Wasserstoff, Cyan oder Hydroxy oder
M-B-$R^5$ auch für eine Gruppe der Formeln

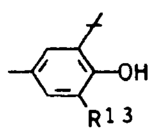 , 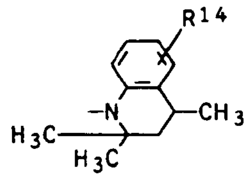 , 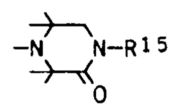 ,

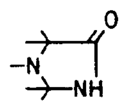 oder 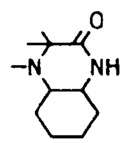 steht, worin

$R^{13}$ $C_1$- bis $C_4$-Alkyl,
$R^{14}$ Wasserstoff, $C_1$- bis $C_4$-Alkyl oder $C_1$- bis $C_4$-Alkoxy und
$R^{15}$ $C_1$- bis $C_{12}$-Alkyl bedeuten.

2. Heterocyclen gemäß Anspruch 1, dadurch gekennzeichnet, daß $R^7$, $R^8$, $R^9$ und $R^{10}$ Methyl sind.

3. Heterocyclen gemäß Anspruch 1 oder 2, dadurch gekennzeichnet, daß $R^{11}$ Wasserstoff ist.

4. Heterocyclen gemäß den Ansprüchen 1 bis 3, dadurch gekennzeichnet, daß B für eine chemische Bindung, für $-CH_2-$ oder $-CH_2-CH_2-$ steht.

5. Verwendung der Heterocyclen gemäß Anspruch 1 bis 4 zum Stabilisieren von organischem Material.

6. Verwendung der Heterocyclen gemäß Anspruch 1 bis 4 zum Stabilisieren von Kunststoffen und Lacken.

7. Verwendung der Heterocyclen gemäß Anspruch 1 bis 4 zum Stabilisieren von Polyurethanen, Polyolefinen oder Polyamiden.

8. Verwendung der Heterocyclen gemäß Anspruch 1 bis 4 als Metallionendesaktivator.

9. Stabilisiertes organisches Material, enthaltend Heterocyclen gemäß den Ansprüchen 1 bis 4.

## Claims

1. A heterocycle of the general formula (I)

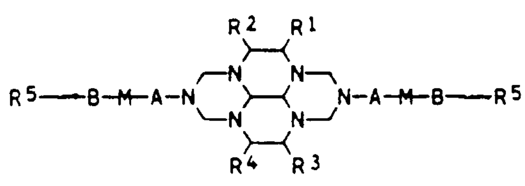

(I)

where

$R^1$, $R^2$, $R^3$ and $R^4$ are each independently of the others hydrogen, $C_1$-$C_{22}$-alkyl or $C_5$- or $C_6$-cycloalkyl or $R^1$ and $R^2$ and also $R^3$ and $R^4$ are each pairwise trimethylene or tetramethylene,
A is a direct bond, methylene or ethylene of the formula $-CH_2-CH_2-$
M is a group of the formula

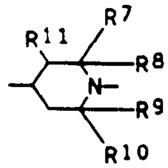

which may be bonded to A not only via the nitrogen but also via the carbon, and where
$R^7$, $R^8$, $R^9$ and $R^{10}$ are each independently of the others $C_1$-$C_4$-alkyl or
$R^7$ and $R^8$ or $R^9$ and $R^{10}$ are pairwise tetramethylene or pentamethylene and
$R^{11}$ is hydrogen or $C_1$-$C_4$-alkyl,
B is a direct bond, $C_1$-$C_{22}$-alkylene, $C_7$-$C_{16}$-phenyl-alkylene or carbonyl-, carboxamido- or carboxylato-interrupted $C_3$-$C_{22}$-alkylene and
$R^5$ is hydrogen, cyano or hydroxyl or M-B-$R^5$ is also a group of the formula

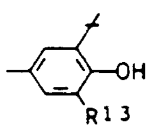 , 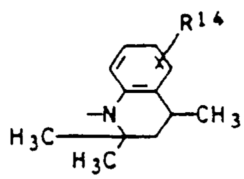 , 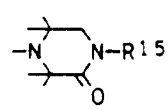 ,

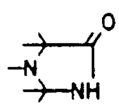 or 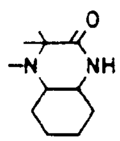 where

$R^{13}$ is $C_1$-$C_4$-alkyl,
$R^{14}$ is hydrogen, $C_1$-$C_4$-alkyl or $C_1$-$C_4$-alkoxy and
$R^{15}$ is $C_1$-$C_{12}$-alkyl.

2. A heterocycle as claimed in claim 1, wherein $R^7$, $R^8$, $R^9$ and $R^{10}$ are each methyl.
3. A heterocycle as claimed in claim 1 or 2, wherein $R^{11}$ is hydrogen.
4. A heterocycle as claimed in any of claims 1 to 3, wherein B is a chemical bond, $-CH_2-$ or $-CH_2-CH_2-$.
5. Use of a heterocycle as claimed in any of claims 1 to 4 for stabilizing organic material.
6. Use of a heterocycle as claimed in any of claims 1 to 4 for stabilizing plastics and paints.
7. Use of a heterocycle as claimed in any of claims 1 to 4 for stabilizing polyurethanes, polyolefins or polyamides.
8. Use of a heterocycle as claimed in any of claims 1 to 4 as metal ion deactivator.
9. Stabilized organic material containing a heterocycle as claimed in any of claims 1 to 4.

## Revendications

1. Hétérocycles de formule générale (I)

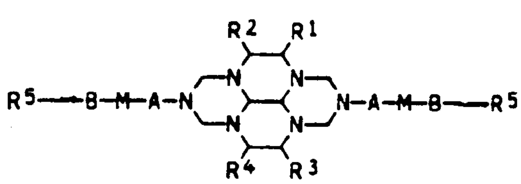 (I),

dans laquelle
$R^1$, $R^2$, $R^3$ et $R^4$ sont mis, indépendamment les uns des autres, pour des atomes d'hydrogène ou pour des restes allyle en $C_1$ à $C_{22}$ ou cycloalkyle en $C_5$ ou $C_6$ ou
$R^1$ et $R^2$ d'une part et $R^3$ et $R^4$ d'autre part sont mis ensemble pour un groupement tri- ou tétraméthylène,
A est mis pour une liaison directe ou pour un reste méthylène ou éthylène de formule $-CH_2-CH_2-$,
M est mis pour un groupement de formule

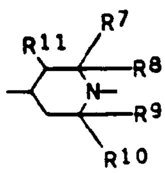 ,

M pouvant être relié à A, aussi bien par l'atome d'azote que par l'atome de carbone,
$R^7$, $R^8$, $R^9$ et $R^{10}$ représentant, indépendamment les uns des autres, des restes allyle en $C_1$ à $C_4$ ou
$R^7$ et $R^8$ et/ou $R^9$ et $R^{10}$ représentant ensemble un groupement tétra- ou pentaméthylène, et
$R^{11}$ représentant un atome d'hydrogène ou un reste allyle en $C_1$ à $C_4$,
B est mis pour une liaison directe, un groupement alkylène en $C_1$ à $C_{22}$, un groupement phénylallylène en $C_7$ à $C_{16}$ ou pour un groupement allylène en $C_3$ à $C_{22}$ interrompu par un reste carbonyle, carboxamide ou ester carboxylique, et
$R^5$ est mis pour un atome d'hydrogène, un groupement cyano ou hydroxy, ou encore
M-B-$R^5$ est mis pour un groupement de formule

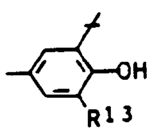 , 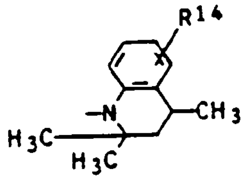 , 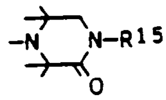 ,

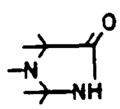 ou 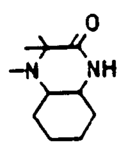

où
$R^{13}$ représente un reste alkyle en $C_1$ à $C_4$,
$R^{14}$ représente un atome d'hydrogène, un reste allyle en $C_1$ à $C_4$ ou alcoxy en $C_1$ à $C_4$, et
$R^{15}$ représente un reste alkyle en $C_1$ à $C_{12}$.

2. Hétérocycles selon la revendication 1, caractérisés en ce que $R^7$, $R^8$, $R^9$ et $R^{10}$ sont des restes méthyle.

3. Hétérocycles selon la revendication 1 ou 2, caractérisés en ce que $R^{11}$ est un atome d'hydrogène.

4. Hétérocycles selon l'une quelconque des revendications 1 à 3, caractérisés en ce que B est mis pour une liaison chimique, pour $-CH_2-$ ou pour $-CH_2-CH_2-$.

5. Utilisation des hétérocycles selon l'une quelconque des revendications 1 à 4 pour la stabilisation de matériaux organiques.

6. Utilisation des hétérocycles selon l'une quelconque des revendications 1 à 4 pour la stabilisation de matières plastiques et de laques.

7. Utilisation des hétérocycles selon l'une quelconque des revendications 1 à 4 pour la stabilisation de polyuréthanes, de polyoléfines ou de polyamides.

8. Utilisation des hétérocycles selon l'une quelconque des revendications 1 à 4 comme désactivateur d'ions métalliques.

9. matériau organique stabilisé, contenant des hétérocycles selon l'une quelconque des revendications 1 à 4.